# FASCICULE DE BREVET EUROPEEN

(11) **EP 0 679 384 B1**
(45) Date de publication et mention de la délivrance du brevet: **18.10.2000**
(21) Numéro de dépôt: 95400441.2
(22) Date de dépôt: 01.03.1995
(51) Int. Cl.: A61K 7/043

(54) **Compositions cosmétiques pour les ongles**
Kosmetische Mittel für Nägeln
Cosmetic compositions for nails

(30) Priorité: 07.04.1994 FR 9404114
(43) Date de publication de la demande: 02.11.1995
(73) Titulaire: L'OREAL, 75008 Paris (FR)
(72) Inventeur: Ramin, Roland, F-91760 Itteville (FR); Mondet, Jean, F-93700 Drancy (FR)
(74) Mandataire: Dodin, Catherine

(56) Documents cités:
- EP-A- 0 226 984
- EP-A- 0 298 271
- FR-A- 2 397 186
- GB-A- 1 032 367

## Description

La présente invention a trait à une composition cosmétique à appliquer sur les ongles, ladite composition comprenant une dispersion aqueuse de polymère.

Il est connu que certaines dispersions aqueuses de polymères particuliers présentent des propriétés filmogènes intéressantes.
Ceci est en particulier connu dans le domaine des produits de maquillage et/ou de soin pour les ongles, ainsi que le montrent, par exemple, les demandes de brevet JP 4103514, EP 143480 ou EP 418469, qui décrivent l'utilisation d'une dispersion aqueuse de polymère comme vernis à ongles ou base de vernis à ongles. Selon les cas, lesdits vernis peuvent être retirés par pelage ou à l'aide d'un solvant organique, ce qui peut entraîner une altération de la surface extérieure de l'ongle d'origine mécanique, dans le cas du pelage, ou d'origine chimique, dans le cas de l'utilisation d'un solvant.
De plus, lorsque l'on souhaite traiter l'ongle, par exemple à l'aide d'un agent protecteur ou durcisseur, le fait de retirer la base contenant cet agent avec un démaquillant contenant un solvant organique peut diminuer les avantages procurés par le traitement.
La demanderesse s'est posée le problème de pallier les inconvénients de l'art antérieur et de proposer une composition cosmétique susceptible d'être appliquée sur l'ongle, pouvant se retirer autrement qu'en utilisant un solvant organique.
De manière surprenante, il a été constaté que si l'on choisissait une dispersion aqueuse adéquate, en association avec un agent plastifiant adéquat, l'on obtenait une composition pouvant s'éliminer par simple lavage à l'eau tout en conservant d'excellentes propriétés filmogènes à température ambiante.

Un objet de la présente invention est donc une composition cosmétique à appliquer sur l'ongle, comprenant une dispersion aqueuse de particules d'au moins un polymère filmogène radicalaire à fonctions acides carboxyliques neutralisées à l'aide d'un agent neutralisant monobasique non volatil à un taux de neutralisation de 10-80%, et d'au moins un agent plastifiant apte à permettre, après application de la dispersion et séchage du film, l'obtention d'un film d'énergie de surface de 30-55 mJ/m² et de polarité 0-0,34.

La composition cosmétique selon l'invention présente l'avantage d'être donc facilement applicable sur toute la surface de l'ongle et de s'éliminer facilement et complètement par simple lavage des mains à l'eau, sans avoir à utiliser de démaquillant, ni même d'eau chaude et/ou d'eau fortement savonneuse.

De plus, la composition cosmétique selon l'invention ayant pour solvant l'eau, il est possible d'y introduire des agents actifs, dont la plupart est hydrosoluble, par exemple de manière à protéger, durcir et/ou soigner l'ongle.
On obtient ainsi un vernis-soin des ongles aqueux, qui, bien qu'appliqué en une seule couche, se présente sous forme d'un film suffisamment épais pour pouvoir remplir ses fonctions à savoir isoler convenablement les agents actifs déposés sur l'ongle de l'extérieur.
Enfin, l'utilisation d'un polymère en dispersion permet l'obtention d'une composition de viscosité adéquate, ce qui n'est pas le cas si l'on utilise un polymère soluble dans l'eau avec lequel la viscosité de la solution obtenue, pour une concentration de 25%, est trop importante pour permettre une application correcte sur l'ongle.

Dans la suite de la présente description, les pourcentages sont donnés en poids sauf indication contraire.

La dispersion aqueuse selon l'invention est préparée selon le procédé usuel de préparation de telles dispersions.
Ce procédé consiste à dissoudre un polymère insoluble dans l'eau dans un solvant organique, à mélanger la solution ainsi obtenue avec de l'eau de manière à former une émulsion, puis à procéder ensuite à l'élimination du solvant organique par évaporation sous pression réduite, de manière à obtenir une dispersion aqueuse constituée de particules du polymère dont la taille est généralement inférieure au µm.

Selon la présente invention, les polymères filmogènes à fonctions acides carboxyliques considérés, sont des polymères synthétiques ayant de préférence un poids moléculaire moyen compris entre 5 000 et 700 000.
Parmi ces polymères filmogènes, on peut notamment citer :
- les copolymères acétate de vinyle/acide crotonique polyoxyéthylénés,
- le copolymère acétate de vinyle/acide crotonique (90/10),
- les terpolymères acétate de vinyle/acide crotonique/néodécanoate de vinyle,
- les copolymères N-octylacrylamide/méthacrylate de méthyle/méthacrylate d'hydroxypropyle/acide acrylique/méthacrylate de tert-butylaminoéthyle,
- le copolymère alterné méthylvinyléther/anhydride maléique (50/50) monoestérifié par le butanol,
- les terpolymères acide acrylique/acrylate d'éthyle/N-tert-butylacrylamide, et
- les polymères répondant à la formule générale suivante: 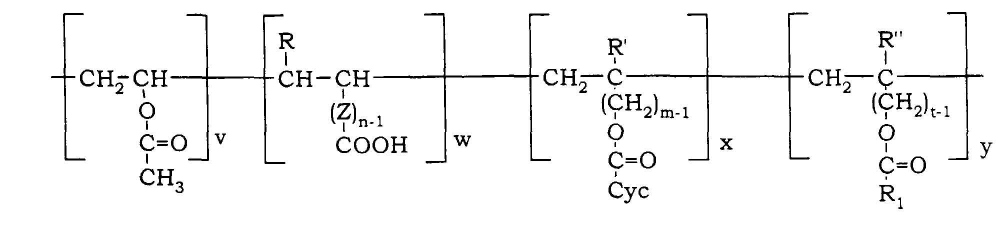 dans laquelle :
- R, R' et R", identiques ou différents, représentent un atome d'hydrogène ou un radical méthyle,
- m, n et t sont 1 ou 2,
- R1 représente un radical alkyle, linéaire ou ramifié, saturé ou insaturé ayant de 2 à 21 atomes de carbone,
- Z représente un radical divalent pris dans le groupe constitué par :

   -CH2-, -CH2-O-CH2- et -CH2-O-(CH2)2-,
- Cyc représente un radical choisi parmi 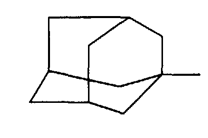 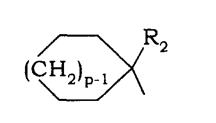 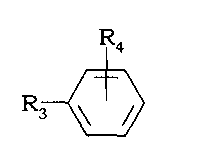 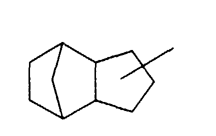 dans lesquels
   - R2 représente un atome d'hydrogène ou un radical méthyle,
   - R3 représente un atome d'hydrogène, un radical méthyle, éthyle, tert-butyle, éthoxy, butoxy ou dodécyloxy,
   - R4 représente un atome d'hydrogène, un radical alkyle ayant de 1 à 4 atomes de carbone ou un radical alkoxy ayant de 1 à 4 atomes de carbone,
   - p est égal à 1 ou 2
   - v représente de 10 à 91% et de préférence de 36 à 84% en poids,
   - w représente de 3 à 20% et de préférence de 6 à 12% en poids,
   - x représente de 4 à 60% et de préférence de 6 à 40% en poids,
   - y représente de 0 à 40% et de préférence de 4 à 30% en poids,
   - v + w + x + y étant égal à 100%.

Le solvant organique utilisé doit être un solvant volatil ou un mélange de tels solvants'présentant un point d'ébullition inférieur à celui de l'eau et être miscible ou partiellement miscible à l'eau.
On peut par exemple utiliser comme solvant l'acétone, la méthyléthylcétone, le tétrahydrofuranne, l'acétate de méthyle, l'acétate d'éthyle, l'isopropanol et/ou l'éthanol.

Lors de la préparation des dispersions aqueuses utilisables dans les compositions selon l'invention, on neutralise partiellement les fonctions acides carboxyliques des polymères filmogènes à un taux allant de 10 % à 80 % .
Cette neutralisation peut être réalisée par addition d'un agent neutralisant dans la solution du polymère dans le solvant organique, avant formation de l'émulsion. Elle peut également être réalisée directement lors de la formation de l'émulsion, en ajoutant l'agent neutralisant dans la phase aqueuse.
Le taux de neutralisation des polymères filmogènes à fonctions acides carboxyliques doit être déterminé de telle sorte qu'ils restent insolubles dans l'eau tout en étant solubles dans le solvant organique.
Les taux limites inférieur et supérieur de neutralisation qu'il convient de ne pas dépasser pour que le polymère reste insoluble dans l'eau sont fonctions de la nature de chaque polymère et sont aisément déterminables par l'homme du métier sur la base de ses connaissances techniques générales.
De façon générale, le taux de neutralisation est compris entre 30 et 80%, si le polymère a moins de 2meq/g de fonctions acides carboxyliques, et entre 10 et 50% si le polymère a plus de 2meq/g de fonctions acides carboxyliques.
La neutralisation des fonctions acides carbcxyliques est réalisée à l'aide d'un composé monobasique non volatil, tel qu'une base minérale comme la soude ou la potasse, ou un aminoalcool par exemple pris dans le groupe constitué par l'amino-2-méthyl-2-propanol-1 (AMP), la triéthanolamine, la triisopropanolamine (TIPA), la monoéthanolamine, la diéthanolamine, la tri-[hydroxy-2-propyl-1]-amine, l'amino-2-méthyl-2-propanediol-1,3 (AMPD) et l'amino-2-hydroxyméthyl-2-propanediol-1,3.

Après neutralisation partielle du polymère, on prépare une émulsion en versant sous agitation, à la solution organique obtenue, une quantité appropriée d'eau contenant éventuellement un agent anti-mousse permettant de faciliter l'évaporation ultérieure du solvant organique.
L'émulsion ainsi obtenue est particulièrement stable sans qu'il soit nécessaire d'employer un agent tensioactif dans la mesure où les groupes carboxylates du polymère se placent à l'interface avec l'eau et protègent les gouttelettes de la coalescence par répulsion électrostatique.
Après formation de l'émulsion, de préférence à une température comprise entre la température ambiante et 70°C, on procède alors à l'évaporation sous pression réduite du solvant organique jusqu'à son élimination totale, l'évaporation étant de préférence réalisée sous léger chauffage.

On obtient ainsi une dispersion aqueuse de particules du polymère filmogène, qui est exempte de tout tensioactif ou autre stabilisant hydrophile tout en étant très stable.
La taille moyenne des particules est généralement comprise entre 10 et 300 nm, mais est de préférence inférieure à 250 nm, avec une polydispersité en taille des particules relativement faible.

Afin d'obtenir une dispersion aqueuse utilisable dans une composition cosmétique selon l'invention, on y introduit au moins un agent plastifiant.
Cet agent plastifiant peut être hydrophile ou hydrophobe. Il est choisi de manière à permettre, en association avec le polymère considéré, un film parfaitement éliminable à l'eau par simple lavage des mains.
Il est également choisi de manière à permettre, après application de la dispersion et séchage du film, l'obtention d'un film d'énergie de surface de 30-55 mJ/m² et de polarité de 0 à 0,34, de préférence inférieure à 0,25.
On peut introduire l'agent plastifiant en mélange dans le solvant organique, lors de la préparation de la dispersion aqueuse, notamment lorsqu'il est du type hydrophobe.
Lorsqu'il est du type hydrophile, on peut l'introduire directement dans la dispersion après sa formation.

Parmi les agents plastifiants pouvant être utilisés dans la présente invention, on peut citer:
- les Carbitols de la Société Union Carbide à savoir le Carbitol ou diéthylène glycol éthyléther, le méthyl Carbitol ou diéthylène glycol méthyléther, le butyl Carbitol ou diéthylène glycol butyléther ou encore l'hexyl Carbitol ou diéthylène glycol hexyléther,
- les Cellosolves de la Société Union Carbide à savoir le Cellosolve ou éthylène glycol éthyléther, le butyl Cellosolve ou éthylène glycol butyléther, l'hexyl Cellosolve ou éthylène glycol hexyléther,
- les dérivés de propylène glycol et en particulier le propylène glycol phényléther, le propylène glycol diacétate, le dipropylène glycol butyléther, le tripropylène glycol butyléther, ainsi que les Dowanols de la Société Dow Chemical tels que le Dowanol PM ou propylène glycol méthyléther, le Dowanol DPM ou dipropylène glycol éthyléther, le Dowanol TPM ou tripropylène glycol méthyléther et le Dowanol DM ou diéthylène glycol méthyléther,
- l'alcool benzylique,
- le citrate de triéthyle,
- le 1,3-butylène glycol,
- les phtalates et adipates de diéthyle, de dibutyle et de diisopropyle,
- les tartrates de diéthyle et de dibutyle,
- les phosphates de diéthyle, de dibutyle et de diéthyl-2-hexyle, et
- les esters de glycérol tels que le diacétate de glycérol (diacétine) et le triacétate de glycérol (triacétine).
On introduit généralement 5 à 40 % d'agent plastifiant par rapport au poids de matière sèche de polymère filmogène neutralisé, ledit agent étant distribué selon son coefficient de partage entre lesdites particules et la phase aqueuse.

On obtient ainsi une dispersion aqueuse selon l'invention, dans laquelle la concentration en poids du polymère filmogène sous forme de particules en dispersion est généralement comprise entre 5 et 50% par rapport au poids total de la dispersion.
La viscosité de ladite dispersion, à une concentration de 25%, est de préférence comprise entre 10 et 3000 mPa.s (mesurée à 25°C).

Les compositions cosmétiques selon l'invention s'éliminent facilement par simple lavage des mains à l'eau.
Elles peuvent être utilisées comme sous-couche d'un vernis à ongles à solvant classique, ou éventuellement comme vernis à élimination facile.

Lorsque ladite composition est utilisée comme sous-couche de vernis solvant, on constate que l'ongle n'a pas tendance à se teinter lors de l'application ultérieure du vernis à solvant, comme cela est le cas dans l'état de la technique; ceci étant du au fait que ledit ongle n'est pas en contact direct avec ledit vernis.
Lorsque l'on introduit des pigments dans lesdites compositions, elles peuvent être utilisées comme vernis.
Enfin, il est également possible d'introduire dans ces compositions cosmétiques, en solution ou en dispersion, des agents actifs tels que des agents protecteurs, durcisseurs et/ou soins de l'ongle, de manière à obtenir une base de soin pour l'ongle, qui peut être ultérieurement recouverte de vernis classique.
Dans le mode particulier d'utilisation comme soin des ongles, on peut imaginer de déposer la base contenant les agents actifs sur l'ongle le soir, ce qui permet le traitement de l'ongle durant la nuit, puis une élimination de cette base le matin par simple lavage des mains.
Parmi les agents actifs utilisables, on peut citer les vitamines et leurs dérivés; les matières premières d'origine biologique et leurs dérivés telles que la kératine, les protéines, les hydrolysats, le chitosane, la mélanine, les oligo-éléments, le collagène; la glycérine; les phospholipides; l'urée; le formal.

L'invention est illustrée plus en détail dans les exemples suivants.

### Exemple 1 : Préparation d'une dispersion aqueuse de copolymère acétate de vinyle/acide crotonique/tert-butyl-4-benzoate de vinyle (65/10/25)

La préparation de ce copolymère est décrite à l'exemple 19 du brevet français n° 78.30596 (FR 2.439.798) et permet l'obtention d'un copolymère se présentant sous forme de billes ayant un diamètre de 0,5 à 1 mm.
On ajoute 100 g du copolymère défini ci-dessus (Indice d'acide : 65), sous agitation, à une solution contenant 275 g d'acétone et 25 g d'adipate diisopropyle.
Après agitation à température ambiante pendant 30 minutes, la dissolution du copolymère est totale.
A la phase organique ainsi obtenue, on ajoute en 5 minutes environ, à température ambiante et sous agitation à l'aide d'un disperseur cisaillant du type Ultra-Turrax à 2000 tr/min, une phase aqueuse pour réaliser l'émulsion.
Ladite phase aqueuse est constituée de 260,55 g d'eau permutée, 1,14 g d'un agent anti-mousse siliconé et de 6,21 g d'amino-2 méthyl-2 propanol-1 (quantité correspondante à 60% de neutralisation d'après l'indice d'acide).
On poursuit l'agitation pendant 10 à 15 min, ce qui permet de conduire à l'obtention d'une émulsion translucide et stable.
On procède alors à la concentration à l'aide d'un évaporateur rotatif sous vide partiel à une température inférieure à 45°C.
Après élimination totale de l'acétone on obtient une dispersion stable laiteuse et peu visqueuse présentant les caractéristiques suivantes:
- concentration du polymère dans la dispersion : 25%
- taille des particules : 58 nm (mesurée en diffusion quasi-élastique de lumière au Coulter modèle M4)
- polydispersité : 0,3
- énergie de surface du film : 50 mJ/m²
- polarité : 0,24

### Exemple 2 : préparation d'une dispersion aqueuse de copolymère acétate de vi-nyle/acide crotonique/néodécanoate de vinyle (résine 28-2930, National Starch)

On ajoute 60 g du copolymère défini ci-dessus (Indice d'acide : 65), sous agitation, à une solution contenant 231 g de méthyléthylcétone, 3 g d'amino-2 méthyl-2 propanol-1 (quantité correspondante à 50% de neutralisation d'après l'indice d'acide) et 12 g d'adipate diisopropyle.
Après agitation à température ambiante pendant 30 minutes, la dissolution du copolymère est totale.
A la phase organique ainsi obtenue, on ajoute, à 60°C et sous agitation à l'aide d'un disperseur cisaillant du type Ultra-Turrax à 2000 tr/min, une phase aqueuse pour réaliser l'émulsion, ladite phase aqueuse étant constituée de 331 g d'eau permutée et 0,69 g d'un agent anti-mousse siliconé.
On poursuit l'agitation pendant 10 à 15 min, ce qui permet de conduire à l'obtention d'une émulsion translucide et stable.
On procède alors à la concentration à l'aide d'un évaporateur rotatif sous vide partiel à une température inférieure à 45°C.
Après élimination totale de l'acétone, on obtient une dispersion stable laiteuse et peu visqueuse présentant les caractéristiques suivantes:
- concentration du polymère dans la dispersion : 15%
- taille des particules : 200 nm (mesurée en diffusion quasi-élastique de lumière au Coulter modèle M4)
- polydispersité : 0,28
- énergie de surface du film : 30 mJ/m²
- polarité : 0

### Exemple 3 : soin des ongles

On prépare la composition suivante, par simple mélange des constituants et agitation:
. dispersion aqueuse de l'exemple 1 96,8985 g
. glycérine 3 g
. glutamate de magnésium 0,0005 g
. glutamate de cuivre 0,0005 g
. glutamate de manganèse 0,0005 g
. protéine de soja 0,1 g

Cette composition est facilement applicable sur l'ongle et sèche en environ 5-10 minutes. On obtient ainsi un film brillant qui s'élimine facilement à l'eau et permet donc d'éviter l'emploi d'un solvant organique.
L'application quotidienne de cette composition sur l'ongle permet d'en améliorer l'aspect général.

### Exemple 4

On prépare la composition suivante, par mélange de la dispersion aqueuse, de la glycérine et du formaldéhyde, suivie d'une faible agitation puis d'une mise en dispersion de l'hydroxypropylcellulose dans la solution obtenue:
. dispersion aqueuse selon l'exemple 2 82,3 g
. eau 15 g
. glycérine 2 g
. formaldéhyde 0,5 g
. hydroxypropylcellulose 0,2 g

On obtient ainsi une composition facilement applicable sur l'ongle et qui donne après séchage, un film brillant qui s'élimine facilement à l'eau.
L'application quotidienne de cette composition sur l'ongle permet, après plusieurs semaines d'application, d'obtenir un durcissement conséquent des ongles mous.

## Revendications

1. Composition cosmétique à appliquer sur l'ongle, comprenant une dispersion aqueuse de particules d'au moins un polymère filmogène radicalaire à fonctions acides carboxyliques neutralisées à l'aide d'un agent neutralisant monobasique non volatil à un taux de neutralisation de 10-80%, et d'au moins un agent plastifiant apte à permettre, après application de la dispersion et séchage du film, l'obtention d'un film d'énergie de surface de 30-55 mJ/m² et de polarité 0-0,34.

2. Composition selon la revendication 1, dans laquelle le polymère filmogène a un poids moléculaire moyen de 5 000 à 700 000 et est choisi dans le groupe constitué par :
- les copolymères acétate de vinyle/acide crotonique polyoxyéthylénés,
- le copolymère acétate de vinyle/acide crotonique (90/10),
- les terpolymères acétate de vinyle/acide crotonique/néodécanoate de vinyle,
- les copolymères N-octylacrylamide/méthacrylate de méthyle/méthacrylate d'hydroxypropyle/acide acrylique/méthacrylate de tert-butylaminoéthyle,
- le copolymère alterné méthylvinyléther/anhydride maléique (50/50) monoestérifié par le butanol,
- les terpolymères acide acrylique/acrylate d'éthyle/N-tert-butylacrylamide, et
- les polymères répondant à la formule générale suivante 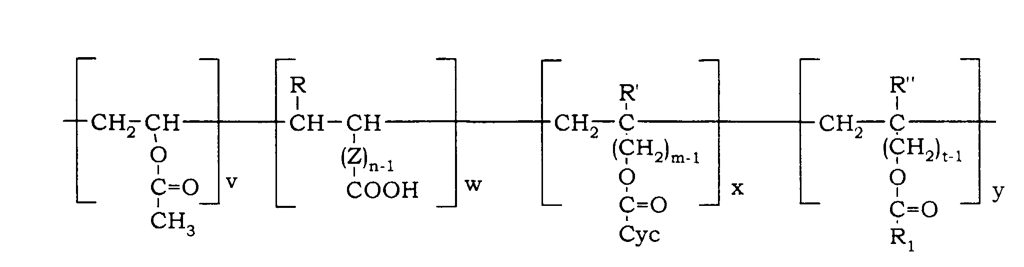 dans laquelle :
- R, R' et R", identiques ou différents, représentent un atome d'hydrogène ou un radical méthyle,
- m, n et t sont 1 ou 2,
- R1 représente un radical alkyle, linéaire ou ramifié, saturé ou insaturé ayant de 2 à 21 atomes de carbone,
- Z représente un radical divalent pris dans le groupe constitué par :
-CH2-, -CH2-O-CH2- et -CH2-O-(CH2)2-,
- Cyc représente un radical choisi parmi 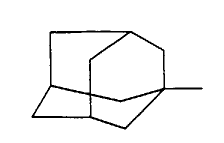 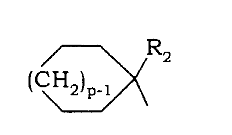 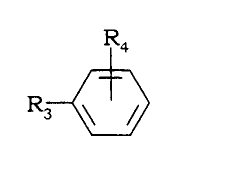 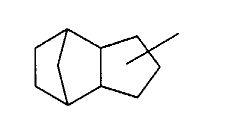 dans lesquels
- R2 représente un atome d'hydrogène ou un radical méthyle,
- R3 représente un atome d'hydrogène, un radical méthyle, éthyle, tert-butyle, éthoxy, butoxy ou dodécyloxy,
- R4 représente un atome d'hydrogène, un radical alkyle ayant de 1 à 4 atomes de carbone ou un radical alkoxy ayant de 1 à 4 atomes de carbone,
- p est égal à 1 ou 2
- v représente de 10 à 91% et de préférence de 36 à 84% en poids,
- w représente de 3 à 20% et de préférence de 6 à 12% en poids,
- x représente de 4 à 60% et de préférence de 6 à 40% en poids,
- y représente de 0 à 40% et de préférence de 4 à 30% en poids,
- v + w + x + y étant égal à 100%.

3. Composition selon la revendication 1, dans laquelle l'agent plastifiant est présent en une proportion de 5-40% en poids par rapport au poids de matière sèche de polymère filmogène neutralisé.

4. Composition selon la revendication 1, dans laquelle la dispersion aqueuse contient en outre au moins un agent actif choisi parmi les vitamines et leurs dérivés; les matières premières d'origine biologiques, la glycérine, les phospholipides, l'urée et le formal.

5. Composition selon la revendication 1, dans laquelle la dispersion aqueuse contient en outre des pigments.

6. Composition selon l'une quelconque des revendications précédentes, caractérisée par le fait que lorsque le polymère filmogène a moins de 2meq/g de fonctions acides carboxyliques, le taux de neutralisation est compris entre 30 et 80%.

7. Composition selon l'une quelconque des revendications précédentes, caractérisée par le fait que lorsque le polymère filmogène a plus de 2meq/g de fonctions acides carboxyliques, le taux de neutralisation est compris entre 10 et 50%.

8. Composition cosmétique selon l'une quelconque des revendications précédentes, caractérisée par le fait que l'agent monobasique non volatil est choisi parmi la soude, la potasse, l'amino-2-méthyl-2-propanol-1, la triéthanolamine, la triisopropanolamine, la monoéthanolamine, la diéthanolamine, la tri-[hydroxy-2-propyl-1]-amine, l'amino-2-méthyl-2-propanediol-1,3 et l'amino-2-hydroxyméthyl-2-propanediol-1,3.

9. Composition cosmétique selon l'une quelconque des revendications précédentes, caractérisée par le fait que l'agent plastifiant est choisi parmi le diéthylène glycol éthyléther, le diéthylène glycol méthyléther, le diéthylène glycol butyléther, le diéthylène glycol hexyléther, l'éthylène glycol éthyléther, l'éthylène glycol butyléther, l'éthylène glycol hexyléther, le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther, l'alcool benzylique, le citrate de triéthyle, le 1,3-butylène glycol, les phtalates et adipates de diéthyle, de dibutyle et de diisopropyle, les tartrates de diéthyle et de dibutyle, les phosphates de diéthyle, de dibutyle et de diéthyl-2-hexyle, et les esters de glycérol.

10. Utilisation d'une composition selon l'une quelconque des revendications 1 à 9 pour l'obtention d'un film éliminable à l'eau.

11. Procédé de soin cosmétique et/ou de maquillage des ongles consistant à appliquer sur les ongles une composition selon l'une quelconque des revendications 1 à 9.

## Patentansprüche

1. Kosmetische, auf die Nägel aufzutragende Zusammensetzung, die eine wäßrige Dispersion enthält, die Partikel aus mindestens einem radikalischen, filmbildenden Polymer mit Carbonsäuregruppen, die mit Hilfe eines nicht flüchtigen einbasigen Neutralisationsmittels neutralisiert sind, wobei der Neutralisationsgrad 10 bis 80 % beträgt, und mindestens einen Weichmacher enthält, der es ermöglicht, nach dem Auftragen der Dispersion und Trocknen des Films einen Film mit einer Oberflächenenergie von 30 bis 55 mJ/m² und einer Polarität von 0 bis 0,34 zu erhalten.

2. Zusammensetzung nach Anspruch 1, wobei das filmbildende Polymer ein mittleres Molekulargewicht von 5 000 bis 700 000 aufweist und ausgewählt ist unter:
- den polyethoxylierten Vinylacetat/Crotonsäure-Copolymeren,
- dem Vinylacetat/Crotonsäure-Copolymer (90/10)
- den Vinylacetat/Crotonsäure/Vinylneodecanoat-Terpolymeren
- den N-Octylacrylamid/Methylmethacrylat/Hydroxypropylmethacrylat/Acrylsäure/*tert.* -Butylaminoethylmethacrylat-Copolymeren,
- dem alternierenden Methylvinylether/Maleinsäureanhydrid-Copolymer (50/50), das mit Butanol einfach verestert ist,
- den Acrylsäure/Ethylacrylat/N-*tert.*-Butylacrylamid-Terpolymeren und
- den Polymeren der folgenden allgemeinen Formel 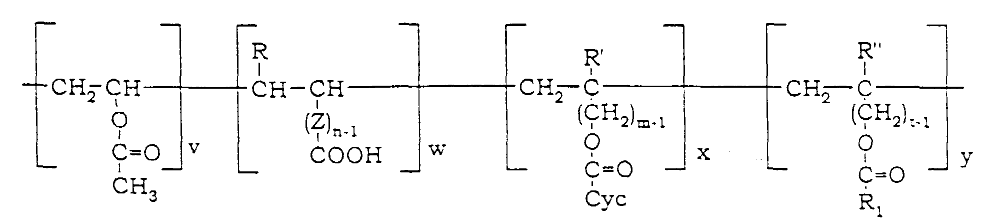 in der bedeuten:
- R, R' und R", die gleich oder verschieden sind, Wasserstoff oder Methyl,
- m, n und t die Zahl 1 oder 2,
- R₁ eine geradkettige oder verzweigte, gesättigte oder ungesättigte Alkylgruppe mit 2 bis 21 Kohlenstoffatomen,
- Z einen zweiwertigen Rest, der unter -CH₂, -CH₂-O-CH₂- und CH₂-O-(CH₂)₂- ausgewählt ist,
- Cyc einen Rest, der ausgewählt ist unter 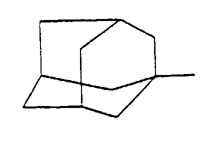 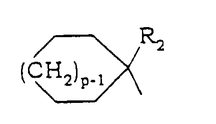 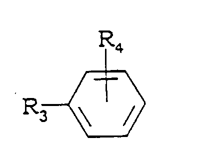 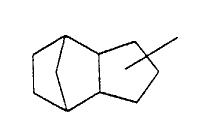 worin bedeuten:
- R₂ Wasserstoff oder Methyl,
- R₃ Wasserstoff, Methyl, Ethyl, *tert.*-Butyl, Ethoxy, Butoxy oder Dodecyloxy,
- R₄ Wasserstoff, eine Alkylgruppe mit 1 bis 4 Kohlenstoffatomen oder eine Alkoxygruppe mit 1 bis 4 Kohlenstoffatomen,
- p die Zahl 1 oder 2,
- v 10 bis 91 Gew.-% und vorzugsweise 36 bis 84 Gew.-%,
- w 3 bis 20 Gew.-% und vorzugsweise 6 bis 12 Gew.-%,
- x 4 bis 60 Gew.-% und vorzugsweise 6 bis 40 Gew.-%,
- y 0 bis 40 Gew.-% und vorzugsweise 4 bis 30 Gew.-%,
- v + w + x + y zusammen 100 Gew.-%.

3. Zusammensetzung nach Anspruch 1, wobei der Weichmacher in einem Anteil von 5-40 Gew.-%, bezogen auf das Gewicht der Trockensubstanz des neutralisierten filmbildenden Polymers, enthalten ist.

4. Zusammensetzung nach Anspruch 1, wobei die wäßrige Dispersion außerdem mindestens einen Wirkstoff enthält, der unter Vitaminen und ihren Derivaten, Ausgangsmaterialien biologischen Ursprungs, Glycerin, Phospholipiden, Harnstoff und Formaldehyd ausgewählt ist.

5. Zusammensetzung nach Anspruch 1, wobei die wäßrige Dispersion außerdem Pigmente enthält.

6. Zusammensetzung nach einem der vorhergehenden Ansprüche, dadurch gekennzeichnet, daß der Neutralisationsgrad 30 bis 80 % beträgt, wenn das filmbildende Polymer weniger als 2 meq/g Carbonsäuregruppen aufweist.

7. Zusammensetzung nach einem der vorhergehenden Ansprüche, dadurch gekennzeichnet, daß der Neutralisationsgrad 10 bis 50 % beträgt, wenn das filmbildende Polymer mehr als als 2 meq/g Carbonsäuregruppen aufweist.

8. Kosmetische Zusammensetzung nach einem der vorhergehenden Ansprüche, dadurch gekennzeichnet, daß das nichtflüchtige, einbasige Mittel unter Natriumhydroxid, Kaliumhydroxid, 2-Amino-2-methyl-1-propanol, Triethanolamin, Triisopropanolamin, Monoethanolamin, Diethanolamin, Tris(2-hydroxy-1-propyl)-amin, 2-Amino-2-methyl-1,3-propandiol und 2-Amino-2-hydroxymethyl-1,3-propandiol und 2-Amino-2-hydroxymethyl-1,3-propandiol ausgewählt ist.

9. Kosmetische Zusammensetzung nach einem der vorhergehenden Ansprüche, dadurch gekennzeichnet, daß der Weichmacher ausgewählt ist unter: Diethylenglykolethylether, Diethylenglykolmethylether, Diethylenglykolbutylether, Diethylenglykolhexylether, Ethylenglykolethylether, Ethylenglykolbutylether, Ethylenglykolhexylether, Propylenglykolphenylether, Propylenglykoldiacetat, Dipropylenglykolbutylether, Tripropylenglykolbutylether, Propylenglykolmethylether, Dipropylenglykolethylether, Tripropylenglykolmethylether, Benzylalkohol, Triethylcitrat, 1,3-Butylenglykol, Diethylphthalat, Dibutylphthalat und Diisopropylphthalat, Diethyladipat, Dibutyladipat und Diisopropyladipat, Diethyltartrat und Dibutyltartrat, Diethylphosphat, Dibutylphosphat und 2-Diethylhexylphosphat, und den Glycerinestern.

10. Verwendung einer Zusammensetzung nach einem der Ansprüche 1 bis 9 für die Herstellung eines Films, der mit Wasser entfernt werden kann.

11. Verfahren zur kosmetischen Pflege und/oder zum Schminken der Nägel, das darin besteht, eine Zusammensetzung nach einem der Ansprüche 1 bis 9 auf die Nägel aufzutragen.

## Claims

1. Cosmetic composition to be applied to the nails, comprising an aqueous dispersion of particles of at least one radical-mediated film-forming polymer containing carboxylic acid functions that are neutralized using a non-volatile monobasic neutralizing agent to a degree of neutralization of 10-80%, and at least one plasticizer which, after applying the dispersion and drying the film, is capable of producing a film with a surface energy of 30-55 mJ/m² and a polarity of 0-0.34.

2. Composition according to Claim 1, in which the film-forming polymer has an average molecular weight of from 5000 to 700,000 and is chosen from the group consisting of:
- polyoxyethylenated vinyl acetate/crotonic acid copolymers,
- vinyl acetate/crotonic acid (90/10) copolymer,
- vinyl acetate/crotonic acid/vinyl neodecanoate terpolymers,
- N-octylacrylamide/methyl methacrylate/hydroxypropyl methacrylate/acrylic acid/tert-butylaminoethyl methacrylate copolymers,
- methyl vinyl ether/maleic anhydride (50/50) alternating copolymer monoesterified with butanol,
- acrylic acid/ethyl acrylate/N-cert-butylacrylamide terpolymers, and
- polymers corresponding to the following general formula 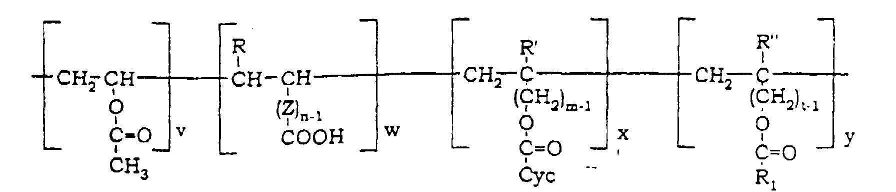 in which:
- R, R' and R", which may be identical or different, represent a hydrogen atom or a methyl radical,
- m, n and t are 1 or 2,
- R1 represents a saturated or unsaturated, linear or branched alkyl radical containing from 2 to 21 carbon atoms,
- Z represents a divalent radical taken from the group consisting of:
-CH₂-, -CH₂-O-CH₂- and -CH₂-O-(CH₂)₂-,
- Cyc represents a radical chosen from 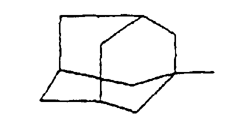 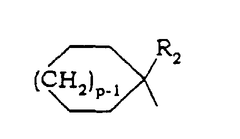 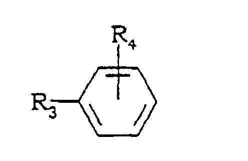 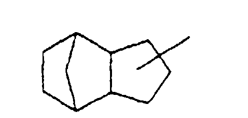 in which
- R₂ represents a hydrogen atom or a methyl radical,
- R₃ represents a hydrogen atom or a methyl, ethyl, tert-butyl, ethoxy, butoxy or dodecyloxy radical,
- R₄ represents a hydrogen atom, an alkyl radical containing from 1 to 4 carbon atoms or an alkoxy radical containing from 1 to 4 carbon atoms,
- p is equal to 1 or 2,
- v represents from 10% to 91% and preferably from 36% to 84% by weight,
- w represents from 3% to 20% and preferably from 6% to 12% by weight,
- x represents from 4% to 60% and preferably from 6% to 40% by weight,
- y represents from 0% to 40% and preferably from 4% to 30% by weight,
- v + w + x + y being equal to 100%.

3. Composition according to Claim 1, in which the plasticizer is present in a proportion of 5-40% by weight relative to the weight of dried neutralized film-forming polymer solids

4. Composition according to Claim 1, in which the aqueous dispersion also contains at least one active agent chosen from vitamins and derivatives thereof; starting materials of biological origin, glycerol, phospholipids, urea and formaldehyde.

5. Composition according to Claim 1, in which the aqueous dispersion also contains pigments.

6. Composition according to any one of the preceding claims, characterized in that, when the film-forming polymer contains less than 2 meq/g of carboxylic acid functions, the degree of neutralization is between 30% and 80%.

7. Composition according to any one of the preceding claims, characterized in that, when the film-forming polymer contains more than 2 meq/g of carboxylic-acid functions, the degree of neutralization is between 10% and 50%.

8. Cosmetic composition according to any one of the preceding claims, characterized in that the non-volatile monobasic agent is chosen from sodium hydroxide, potassium hydroxide, 2-amino-2-methyl-1-propanol, triethanolamine, triisopropanolamine, monoethanolamine, diethanolamine, tris[2-hydroxy-1-propyl]amine, 2-amino-2-methyl-l,3-propanediol and 2-amino-2-hydroxymethyl-1,3-propanediol.

9. Cosmetic composition according to any one of the preceding claims, characterized in that the plasticizer is chosen from diethylene glycol ethyl ether, diethylene glycol methyl ether, diethylene glycol butyl ether, diethylene glycol hexyl ether, ethylene glycol ethyl ether, ethylene glycol butyl ether, ethylene glycol hexyl ether, propylene glycol phenyl ether, propylene glycol diacetate, dipropylene glycol butyl ether, tripropylene glycol butyl ether, propylene glycol methyl ether, dipropylene glycol ethyl ether, tripropylene glycol methyl ether, benzyl alcohol, triethyl citrate, 1,3-butylene glycol, diethyl, dibutyl and diisopropyl phthalates and adipates, diethyl and dibutyl tartrates, diethyl, dibutyl and 2-diethylhexyl phosphates, and glycerol esters.

10. Use of a composition according to any one of Claims 1 to 9, to obtain a film which can be removed with water.

11. Cosmetic care process and/or make-up process for the nails, which consists in applying a composition according to any one of Claims 1 to 9 to the nails.
